# EUROPEAN PATENT SPECIFICATION

(11) **EP 1 988 985 B1**
(45) Date of publication and mention of the grant of the patent: **07.08.2013**
(21) Application number: 05786527.1
(22) Date of filing: 17.08.2005
(51) Int. Cl.: B01F 17/00, A61K 8/46, C11D 1/37, C11D 1/75, A61Q 19/10, A61K 8/40

(54) **STRUCTURED SURFACTANT COMPOSITIONS**
STRUKTURIERTE TENSIDZUSAMMENSETZUNGEN
COMPOSITIONS TENSIOACTIVES STRUCTUREES

(30) Priority: 24.06.2005 US 693672 P
(43) Date of publication of application: 12.11.2008
(73) Proprietor: Rhodia, Inc., Cranbury, NJ 08512 (US)
(72) Inventor: FRANTZ, Seren, Bensalem, PA 19020 (US); SRIDHAR, Bharathy, East Brunswick, NJ 08816 (US); WARBURTON, Stewart, Alexander, West Windsor, NJ 08550 (US)
(74) Representative: Gillard, Richard Edward
(86) International application number: PCT/US2005/029241
(87) International publication number: WO 2007/001341

(56) References cited:
- EP-A1- 1 416 038
- EP-A2- 0 151 884
- WO-A1-2004/056950
- GB-A- 2 272 449
- US-A- 5 039 451
- US-A- 5 776 883
- US-A1- 2005 020 468

## Description

### Field of the Invention

This invention relates to surfactant compositions, more particularly to structured surfactant systems.

### Background of the Invention

Structured surfactant compositions are pumpable compositions that exhibit shear-thinning viscosity and have the capacity physically to suspend water insoluble or partially water soluble ingredients. In many cases, the surfactant is present in such structured surfactant compositions in the form of packed spherulites, i.e., lamellar droplets, formed from an aqueous solution of the surfactant.

Structured surfactant compositions are useful in personal care applications, such as shampoos, body wash, hand soap, lotions, creams, conditioners, shaving products, facial washes, neutralizing shampoos, and skin treatments, in home care applications, such as liquid detergents, laundry detergents, hard surface cleansers, dish wash liquids, toilet bowl cleaners, car cleansers, and in other applications, such as oil field and agrochemical applications.

In some structured liquid compositions, relatively high levels of structurant are needed in order to create a structured system.

What is needed is a structured surfactant composition that provides typical structured surfactant properties, that is, shear-thinning viscosity and a capacity to suspend water insoluble or partially water soluble components, using a lower relative amount of structuring agent.

EP 1 416 038 concerns a liquid hypochlorite bleaching composition which is suitable for use in a toilet rim mounted toilet bowl cleaning device, the composition having a shear thinning rheology and being capable of generating a foam height of at least 3 cm.

EP 0 151 884 concerns a liquid detergent composition suitable for laundry use consisting essentially of water, electrolyte, active ingredient and preferably a builder.

### Summary of the Invention

In a first aspect, the present invention is directed to an aqueous structured surfactant composition, comprising based on 100 parts by weight of the composition:
water,
from greater than 0 to 6 parts by weight of an electrolyte selected from potassium pyrophosphate, potassium tripolyphosphate, sodium citrate, potassium citrate, calcium chloride, calcium bromide, zinc halides, barium chloride, calcium nitrate, alkali metal halides, ammonium halides, alkali metal nitrate, ammonium nitrates, uncapped polyacrylates, polymaleates, polycarboxylates, lignin sulphonates and naphthalene sulphonate formaldehyde copolymers,
from 8 to 40 parts by weight of one or more anionic surfactants selected from linear alkylbenzene sulfonates, alpha olefin sulfonates, paraffin sulfonates, alkyl ester sulfonates, alkyl sulfates, alkyl alkoxy sulfates, alkyl sulfonates, alkyl alkoxy carboxylates, alkyl alkoxylated sulfates, monoalkyl phosphates, dialkyl phosphates, sarcosinates, sulfosuccinates, isethionates, and taurates, and mixtures thereof,
and greater than 0 to 5 parts by weight of one or more amine oxides according to formula (III): 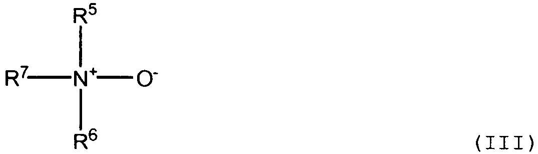
   wherein R⁵, R⁶, and R⁷ are each independently alkyl, hydroxyalkyl, alkoxyl, alkenyl, R⁸-R⁹ , R¹⁰-C(O)-NH-R¹¹, a phosphate moiety, a phosphonate moiety, or any two of R⁵, R⁶, and R⁷are fused to form a 5- to 8-membered saturated or unsaturated heterocyclic ring that includes the nitrogen atom of the amine oxide and, optionally, further includes a second nitrogen atom or an oxygen atom as a ring member and which may be further substituted with alkyl or amino on one or more of the ring atoms,
   R⁸ and R¹⁰ are each independently H, alkyl, or alkenyl,
   R⁹ is alkyleneoxy, and
   R¹¹ is alkylene or alkyleneoxy,
   wherein the amount of amine oxide is from 10 to 50 parts by weight per 100 parts by weight of the anionic surfactant, said composition exhibiting shear-thinning viscosity and is capable of suspending water insoluble or partially water soluble components.

In a second aspect, the present invention is directed to a multi-phase composition comprising:
a first phase that comprises, the aforementioned aqueous structured surfactant composition, and
one or more additional phases that comprise one or more water insoluble or partially water insoluble components and is at least substantially distinct from the first phase.

In a third aspect, the present invention is directed to a method for making the aforementioned aqueous structured surfactant composition.

In a fourth aspect, the aforementioned multi-phase composition is a personal care composition and the one or more water insoluble or partially water insoluble components comprise one or more benefit agents.

The personal care composition of the present invention exhibits structured surfactant properties, that is, shear-thinning viscosity and a capacity to suspend water insoluble or partially water soluble components and typically require a lower total relative amount of structuring agents.

### Detailed Description of Invention and Preferred Embodiments

As used herein in reference to viscosity, the terminology "shear-thinning" means that such viscosity decreases with an increase in shear rate. Shear-thinning may be characterized as a "non-Newtonian" behavior, in that it differs from that of a classical Newtonian fluid, for example, water, in which viscosity is not dependent on shear rate.

As used herein in reference to a component of an aqueous composition, the terminology "water insoluble or partially water soluble components" means that the component is present in the aqueous composition at a concentration above the solubility limit of the component so that, in the case of a water insoluble component, the component remains substantially non-dissolved in the aqueous composition and, in the case of a partially water soluble component, at least a portion of such component remains undissolved in the aqueous composition.

As used herein, characterization of an aqueous composition as "capable of suspending", or as being "able to suspend" water insoluble or partially water insoluble components means that the composition substantially resists flotation of such components in the composition or sinking of such components in such composition so that such components appear to be neutrally buoyant in such composition and remain at least substantially suspended in such composition under the anticipated processing, storage, and use conditions for such aqueous composition.

In one embodiment, the structured surfactant composition comprises at least one lamellar phase, said lamellar phase comprising water, at least a portion of the anionic surfactant and at least a portion of the amine oxide.

As used herein, the terminology "lamellar phase" means a phase that comprises a plurality of bilayers of surfactant arranged in parallel and separated by liquid medium. A lamellar phase is detectable by, for example, small angle x-ray measurement or by evidence of birefringence under a cross-polarized microscope. Lamellar phases include both spherulitic phases and the typical form of the liquid crystal G-phase, as well as mixtures thereof. "G-phases", which are sometimes referred to in the literature a L_{α} phases, are typically pumpable, non-Newtonian, anisotropic products that are cloudy looking and exhibit a characteristic "smeary" appearance on flowing. Lamellar phases, can exist in several different forms, including domains of parallel sheets which constitute the bulk of the typical G-phases described above and spherulites formed from a number of concentric spheroidal shells, each of which is a bilayer of surfactant. In this specification the term "G-phase" will be reserved for compositions which are at least partly of the former type. The spherulites are typically between 0.1 and 50 microns in diameter and so differ fundamentally from micelles. Unlike micellar solutions, spherulitic compositions are typically anisotropic and non-Newtonian. When close packed, spherulites have good solid suspending properties and are capable of suspending water insoluble or partially water soluble solids, liquids and/or gases as a separate, discontinuous phase suspended in a continuous matrix of the surfactant composition.

As used herein, the term "alkyl" means a saturated straight, branched, or cyclic hydrocarbon radical, such as for example, methyl, ethyl, n-propyl, iso-propyl, n-butyl, sec-butyl, t-butyl, pentyl, n-hexyl, cyclohexyl, decyltetradecyl, octadecyloctadecyl.

As used herein, the term "hydroxyalkyl" means an alkyl radical that is substituted with one or more hydroxyl substituents, such as for example, hydroxyethyl, hydroxypropyl.

As used herein, the term "alkoxyl" means a monovalent saturated or unsaturated straight or branched alkyl ether radical, such as for example, ethoxy, propoxy, isopropoxy, butoxy.

As used herein, the term "alkylene" means a bivalent straight or branched acyclic saturated hydrocarbon radical, including methylene and polymethylene, such as, for example, dimethylene, tetramethylene, 2-methyltrimethylene.

As used herein, the term "alkenyl" means an unsaturated straight, branched, or cyclic hydrocarbon radical having at least one carbon-carbon double bond per radical, such as for example, propenyl, butenyl.

As used herein, the term "alkyleneoxy" means a bivalent straight or branched acyclic ether or polyether radical such as, for example, ethyleneoxy, poly(ethyleneoxy), propyleneoxy, poly(propyleneoxy), poly(ethoxylenepropyleneoxy).

As used herein, the term "phosphate moiety" means a phosphorus-containing radical according to formula (I): 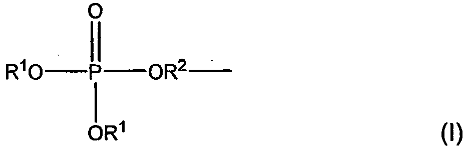 wherein
each R¹ is independently H, alkyl, or alkenyl and
R² is alkylene or alkyleneoxy,
as well as corresponding salt forms, such as: 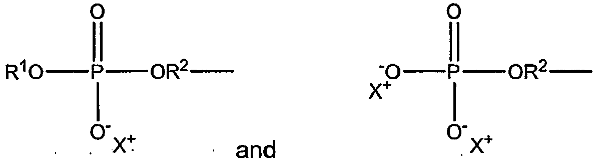 wherein X⁺ is a cation, typically a sodium or potassium cation.

As used herein, the term "phosphonate moiety" means a phosphorus-containing radical according to formula (II): 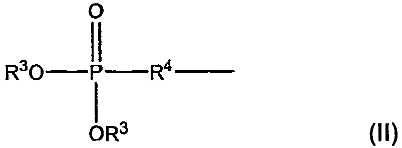 wherein:
each R³ is independently H, alkyl, or alkenyl and
R⁴ is alkylene or alkyleneoxy,
as well as corresponding salt forms, such as: 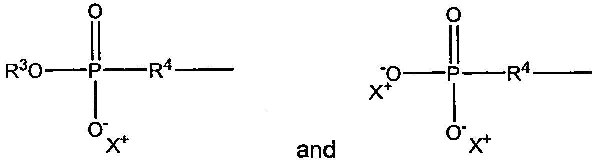 wherein X⁺ is a cation, typically a sodium or potassium cation.

As used herein, the terminology "(Cₙ-Cₘ)" in reference to an organic group, wherein n and m are each integers, indicates that the group may contain from n carbon atoms to m carbon atoms per group.

Anionic surfactants are known. Any anionic surfactant that is acceptable for use in the intended end use application is suitable as the anionic surfactant component of the composition of the present invention, including, for example, linear alkylbenzene sulfonates, alpha olefin sulfonates, paraffin sulfonates, alkyl ester sulfonates, alkyl sulfates, alkyl alkoxy sulfates, alkyl sulfonates, alkyl alkoxy carboxylates, alkyl alkoxylated sulfates, monoalkyl phosphates, dialkyl phosphates, sarcosinates, sulfosuccinates, isethionates, and taurates, as well as mixtures thereof. Commonly used anionic surfactants that are suitable as the anionic surfactant component of the composition of the present invention include, for example, ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium-monoalkyl phosphates, sodium dialkyl phosphates, sodium lauroyl sarcosinate, lauroyl sarcosine, cocoyl sarcosine, ammonium cocyl sulfate, ammonium lauryl sulfate, sodium cocyl sulfate, sodium trideceth sulfate, sodium tridecyl sulfate, ammonium trideceth sulfate, ammonium tridecyl sulfate, sodium cocoyl isethionate, disodium laureth sulfosuccinate, sodium methyl oleoyl taurate, sodium laureth carboxylate, sodium trideceth carboxylate, sodium lauryl sulfate, potassium cocyl sulfate, potassium lauryl sulfate, monoethanolamine cocyl sulfate, sodium tridecyl benzene sulfonate, and sodium dodecyl benzene sulfonate. Branched anionic surfactants are particularly preferred, such as sodium trideceth sulfate, sodium tridecyl sulfate, ammonium trideceth sulfate, ammonium tridecyl sulfate, and sodium trideceth carboxylate.

The cation of any anionic surfactant is typically sodium but may alternatively be potassium, lithium, calcium, magnesium, ammonium, or an alkyl ammonium having up to 6 aliphatic carbon atoms including isopropylammonium, monoethanolammonium, diethanolaillmonium, and triethanolammonium. Ammonium and ethanolammonium salts are generally more soluble that the sodium salts. Mixtures of the above cations may be used.

In one embodiment, the structured surfactant composition of the present invention comprises, based on 100 pbw of the composition, from about 3 to about 40 pbw, more typically from about 5 to about 30 pbw, and still more typically from about 8 to about 20 pbw, of the one or more anionic surfactants.

Amine oxides are known compounds according to formula (III): 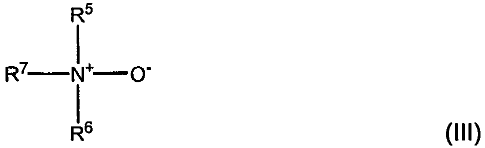 wherein each of R⁵, R⁶, and R⁷ is independently an organic group, which may, optionally, include one or more heteroatoms. Suitable amine oxides include, for example, almondamidopropylamine oxide (N-[3-(dimethylamino)propyl]almond amides-N-oxide), babassuamidopropylamine oxide (N-[3-(dimethylamino)propyl]babassu amides-N-oxide), behenamine oxide (N,N-dimethyl-1-docosanamine-N-oxide), cocamidopropylamine oxide (N-[3-(dimethylamino)propyl]coco amides-N-oxide), coco-morpholine oxide (morphaline, 4-coco alkyl derivs, 4-oxides), decylamine oxide (N,N-dimethyl-1-decylamine-N-oxide), decyltetradecylamine oxide, diaminopyrimidine oxide, dihydroxyethyl C8-10 alkoxypropylamine oxide, dihydroxyethyl C9-11 alkoxypropylamine oxide, dihydroxyethyl C12-15 alkoxypropylamine oxide, dihydroxyethyl cocamine oxide (N,N-bis(2-hydroxyethyl) cocamine oxide), dihydroxyethyl lauramine oxide (N,N-bis(2-hydroxyethyl) lauramine oxide), dihydroxyethyl stearamine oxide (N,N-bis(2-hydroxyethyl) stearamine oxide), dihydroxyethyl tallowamine oxide (N,N-bis(2-hydroxyethyl) tallowamine oxide), hydrogenated palm kernel amine oxide, hydrogentated tallowamine oxide, hydroxyethyl hydroxypropyl C12-15 alkoxypropylamine oxide, isostearamidopropylamine oxide (N-[3-(dimethylamino)propyl] isooctadecanamide-N-oxide), isostearamidopropyl morpholine oxide (N-[3-(4-morpholinyl)propyl] isooctadecanamide-N-oxide), lauramidopropylamine oxide (N-[3-(dimethylamino)propyl] dodecanamide-N-oxide), lauramine oxide (N,N-dimethyl-1-dodecanamine-N-oxide), methyl morpholine oxide, milkamidopropylamine oxide, minkamidopropylamine oxide, myristamidopropylamine oxide (N-[3-(dimethylamino)propyl] tetradecanamide-N-oxide), myristamine oxide (N,N-dimethyl-1-tetradecanamine-N-oxide), myristyl/cetyl amine oxide (N,N-dimethyl-1-myristamine/cetylamine-N-oxide), oleamidopropylamine oxide (N-[3-(dimethylamino)propyl]-9-octadecenamide-N-oxide), oleamine oxide (N,N-dimethyl-9-octadecen-1-Amine-N-oxide), olivamidopropylamine oxide, palmitamidopropylamine oxide, palmitamine oxide, PEG-3 lauramine oxide, potassium dihydroxyethyl cocamine oxide phosphate, potassium trisphosphonomethylamine oxide, sesamidopropylamine oxide, soyamidopropylamine oxide, stearamidopropylamine oxide, stearamine oxide, tallowamidopropylamine oxide, tallowamine oxide, undecylenamidopropylamine oxide, wheat germamidopropylamine oxide, as well as mixtures of such amine oxides.

In one embodiment, the amine oxide component of the composition of the present invention comprises at least one compound according to formula (III), wherein R⁵, R⁶, and R⁷are each independently alkyl, hydroxyalkyl, alkoxyl, alkenyl, R⁸-R⁹-, R¹⁰-C(O) NH-R¹¹ a phosphate moiety, a phosphonate moiety, or any two of R⁵, R⁶, and R⁷are fused to form a 5- to 8-membered saturated or unsaturated heterocyclic ring that includes the nitrogen atom of the amine oxide and, optionally, further includes a second nitrogen atom or an oxygen atom as a ring member and which may be further substituted with alkyl or amino on one or more of the ring atoms,
R⁸ and R¹⁰ are each independently H, alkyl, or alkenyl,
R⁹ is alkyleneoxy, and
R¹¹ is alkylene or alkyleneoxy.

In one embodiment, R⁵, R⁶, and R⁷ are each independently (C₁-C₅₀)alkyl, (C₁-C₅₀)hydroxyalkyl, (C₁-C₅₀)alkoxyl, (C₂-C₅₀)alkenyl, R⁸-R⁹-, -R¹⁰-C(O) -NH-R¹¹ a phosphate moiety, a phosphonate moiety, or any two of R⁵, R⁶, and R⁷ are fused to form a 5- or 6-membered saturated or unsaturated heterocyclic ring that includes the nitrogen atom of the amine oxide and, optionally, further includes a second nitrogen atom or an oxygen atom as a ring member and which may be further substituted with (C₁-C₅₀)alkyl or amino on one or more of the ring atoms,
R⁸ and R¹⁰ are each independently H, (C₁-C₅₀)alkyl, or (C₂-C₅₀)alkenyl,
R⁹ is (C₁-C₅₀)alkyleneoxy, and
R¹¹ is (C₁-C₅₀)alkylene or (C₂-C₅₀)alkyleneoxy.

In one embodiment, at least one of R⁵, R⁶, and R⁷ is (C₁-C₅₀)alkyl, (C₁-C₅₀)hydroxyalkyl, (C₁-C₅₀)alkoxyl, or (C₂-C₅₀)alkenyl.

In one embodiment, at least one of R⁵, R⁶, and R⁷ is derived from almond oil, babassu oil, hydrogenated palm kernel oil, hydrogenated tallow, milk, mink oil, olive oil, palm oil, sesame oil, soy, tallow, or wheat germ oil.

In one embodiment, one of R⁵, R⁶, and R⁷ is (C₈-C₅₀)alkyl or (C₈-C₅₀)alkenyl and the other two of R⁵, R⁶, and R⁷ are each independently (C₁-C₇)alkyl, (C₁-C₇)hydroxyalkyl, (C₁-C₇)alkoxyl.

In one embodiment, at least one of R⁵, R⁶, and R⁷ is R¹⁰-C(O) -NH-R¹¹-, wherein R¹⁰ is (C₁-C₅₀)alkyl or (C₂-C₅₀)alkenyl, and R¹¹ is (C₁-C₂₀)alkylene, typically methylene or (C₂-C₂₀)polymethylene.

In one embodiment, at least one of R⁵, R⁶, and R⁷ is a radical R¹⁰-_{C}(_{O}) NH-R¹¹ wherein R¹⁰ is (C₁-C₅₀)alkyl or (C₂-C₅₀)alkenyl and the R¹⁰-C(O) - portion of the radical is derived from almond oil, babassu oil, hydrogenated palm kernel oil, hydrogenated tallow, milk, mink oil, olive oil, palm oil, sesame oil, soy, tallow, or wheat germ oil, and R¹¹ is (C₁-C₂₀)alkylene, typically methylene or (C₂-C₂₀)polymethylene.

In one embodiment, at least one of R⁵, R⁶, and R⁷ is a phosphate moiety or a phosphonate moiety.

In one embodiment, two of R⁵, R⁶, and R⁷ are fused to form a 5- or 6-membered saturated or unsaturated heterocyclic ring which includes the nitrogen atom of the amine oxide and a second nitrogen atom as ring members and wherein the ring is substituted with amino on one or more of the ring atoms.

In one embodiment, two of R⁵, R⁶, and R⁷ are fused to form a 5- or 6-membered saturated or unsaturated heterocyclic ring which includes the nitrogen atom of the amine oxide and an oxygen atom as ring members and wherein the ring is substituted with alkyl on one or more of the ring carbons.

In one embodiment, the structured surfactant composition of the present invention comprises, based on 100 pbw of the composition, from greater than 0 to about 20 pbw, typically from about 0.5 to 10 pbw, more typically from about 0.75 to about 8 pbw, and still more typically from about 1 to about 6 pbw, still more typically from about 1.5 to about 5 pbw, and even more typically from about 2 to about 5 pbw, amine oxide.

In one embodiment, the structured surfactant composition of the present invention comprises, based on 100 pbw of the composition, from about 5 to about 40 pbw, more typically from about 8 to about 20 pbw, and still more typically from about 10 to about 15 pbw, anionic surfactant selected from linear sulfate anionic surfactants, and from greater than zero to about 20 pbw, more typically from about 0.5 to about 10 pbw, and still more typically from about 1 to about 6 pbw, amine oxide. Suitable linear sulfate anionic surfactants include, for example, linear alkyl sulfates, such as sodium lauryl sulfate, ammonium lauryl sulfate, sodium coco sulfate sodium myristyl sulfate, and linear alkyl alkoxy sulfates, such as sodium laureth sulfate ammonium laureth sulfate, sodium coco ether sulfate.

When present in a sufficient amount relative to the amount of water and anionic surfactant components of the compositions of the present invention, the amine oxide acts as a structurant for the anionic surfactant, that is, as a compound that, in combination with the water and anionic surfactant, forms a shear-thinning fluid that is capable of suspending water insoluble or partially water soluble components. In one embodiment, the amine oxide is present in an amount relative to the amounts of water and anionic surfactant that is at least effective to, in combination with such water and anionic surfactant, form a shear-thinning fluid that is capable of suspending water insoluble or partially water soluble components.

In one embodiment, shear-thinning and component-suspending properties for structured surfactant compositions based on a given anionic surfactant are achieved using a relatively low level of amine oxide as a structurant, compared to the amounts of previously known structurants, such as electrolytes, fatty alcohols or alkanolamides, required to obtain such properties.

In one embodiment, the structured surfactant composition comprises from about 1 to about 70 pbw, more typically from about 5 to about 60 pbw, and still more typically from about 10 to about 50 pbw, amine oxide per 100 pbw anionic surfactant.

In one embodiment, the surfactant blend of the present invention comprises, based on 100 pbw of the blend, from 10 pbw to 75 pbw of one or more anionic surfactants, and from greater than 1 pbw, typically greater than 2 pbw, more typically greater than 3 pbw, even more typically greater than 4 pbw, still more typically greater than 5 pbw, to 30 pbw of one or more amine oxides.

The structured surfactant composition of the present invention may optionally further comprise, in addition to the anionic surfactant and amine oxide components of the composition of the present invention, one or more cationic surfactants, one or more additional non-ionic surfactants, one or more zwitterionic surfactants, one or more amphoteric surfactants, one or more electrolytes, or a mixture thereof. In cases where any of such optional components functions as a structurant for the anionic surfactant, each of such components may independently be present in an amount in excess of the minimum amount effective to act as a structurant. Cationic surfactants and certain non-ionic surfactants, such as such as fatty alcohols, ethoxylated alcohols, and fatty acids, are known to act as structurants for anionic surfactants.

Typically, the greater the amount of anionic surfactant present in relation to its solubility, the lesser the amount of structurant required in order to form a structure capable of supporting solid materials and/or to cause flocculation of the structured surfactant. The structurant is incorporated in an amount sufficient to promote the structured surfactant composition and may be added separately or may be included in one of the other raw materials added to the composition.

In one embodiment, the structured surfactant composition of the present invention comprises, based on 100 pbw of such composition, a total amount of up to about 40 pbw, more typically from about 0.5 to about 25 pbw and still more typically from about 1 to about 10 pbw of one or more structurants, inclusive of the amount of amine oxide.

Cationic surfactants are known. Any cationic surfactant that is acceptable for use in the intended end use application is suitable as the cationic surfactant component of the composition of the present invention, including, for example, cationic surfactants according to formula (IV) below: 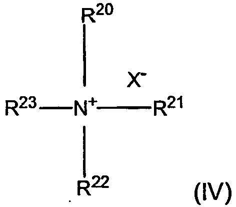 wherein:
R²⁰, R²¹ , R²¹, and R²³ are each independently hydrogen or an organic group, provided that at least one of R²⁰, R²¹, R²², and R²³ is not hydrogen, and
X⁷ is an anion.

If from one to three of the R²⁰, R²¹, R²², and R²³ groups are each hydrogen, then the resulting compound may be referred to as an amine salt. Some examples of cationic amines include polyethoxylated (2) oleyl/stearyl amine, ethoxylated tallow amine, cocoalkylamine, oleylamine, and tallow alkyl amine.

For quaternary ammonium compounds (generally referred to as "quats") R²⁰, R²¹, R²², and R²³ may be the same or different organic group, but may not be hydrogen. In one embodiment, R²⁰, R²¹ , R²², and R²³ are each (C₈-C₂₄) branched or linear hydrocarbon groups which may be substituted or interrupted by additional functional moieties and include, for example, fatty acids or derivatives thereof, including esters of fatty acids and fatty acids with alkoxylated groups, alkyl amido groups, aromatic rings, heterocyclic rings, phosphate groups, epoxy groups, and hydroxyl groups. The nitrogen atom may also be part of a heterocyclic or aromatic ring system, e.g., cetethyl morpholinium ethosulfate or steapyrium chloride.

Suitable anions include, for example, chloride, bromide, methosulfate, ethosulfate, lactate, saccharinate, acetate or phosphate.

Examples of quaternary ammonium compounds of the monoalkyl amine derivative type include: cetyl trimethyl ammonium bromide (also known as CETAB or cetrimonium bromide), cetyl trimethyl ammonium chloride (also known as cetrimonium chloride), myristyl trimethyl ammonium bromide (also known as myrtrimonium bromide or Quaternium-13), stearyl dimethyl benzyl ammonium chloride (also known as stearalkonium chloride), oleyl dimethyl benzyl ammonium chloride, (also known as olealkonium chloride), lauryl/myristryl trimethyl ammonium methosulfate (also known as cocotrimonium methosulfate), cetyl-dimethyl-(2)hydroxyethyl ammonium dihydrogen phosphate (also known as hydroxyethyl cetyldimonium phosphate), bassuamidopropylkonium chloride, cocotrimonium chloride, distearyldimonium chloride, wheat germ-amidopropalkonium chloride, stearyl octyldimonium methosulfate, isostearaminopropal-konium chloride, dihydroxypropyl PEG-5 linoleaminium chloride, PEG-2 stearmonium chloride, Quaternium 18, Quaternium 80, Quaternium 82, Quaternium 84, behentrimonium chloride, dicetyl dimonium chloride, behentrimonium methosulfate, tallow trimonium chloride and behenamidopropyl ethyl dimonium ethosulfate.

Quaternary ammonium compound of the dialkyl amine derivative type distearyldimonium chloride, dicetyl dimonium chloride, stearyl octyldimonium methosulfate, dihydrogenated palmoylethyl hydroxyethylmonium methosulfate, dipalmitoylethyl hydroxyethylmonium methosulfate, dioleoylethyl hydroxyethylmonium methosulfate, hydroxypropyl bisstearyldimonium chloride, and mixtures thereof.

Quaternary ammonium compounds of the imidazoline derivative type include, for example, isostearyl benzylimidonium chloride, cocoyl benzyl hydroxyethyl imidazolinium chloride, cocoyl hydroxyethylimidazolinium PG-chloride phosphate, Quaternium 32, and stearyl hydroxyethylimidonium chloride, and mixtures thereof.

Nonionic surfactants are known. Any nonionic surfactant that is acceptable for use in the intended end use application is suitable as the optional nonionic surfactant component of the composition of the present invention, including compounds produced by the condensation of alkylene oxide groups with an organic hydrophobic compound which may be aliphatic or alkyl aromatic in nature. Examples of useful nonionic surfactants include the polyethylene, polypropylene, and polybutylene oxide condensates of alkyl phenols, fatty acid amide surfactants, polyhydroxy fatty acid amide surfactants, amine oxide surfactants, alkyl ethoxylate surfactants, alkanoyl glucose amide surfactants, and alkylpolyglycosides. Specific examples of suitable nonionic surfactants include alkanolamides such as cocamide DEA, cocamide MEA, cocamide MIPA, lauramide DEA, and lauramide MEA, alkyl amine oxides such as lauramine oxide, cocamine oxide, cocamidopropylamine oxide, and lauramidopropylamine oxide, sorbitan laurate, sorbitan distearate, fatty acids or fatty acid esters such as lauric acid, and isostearic acid, fatty alcohols or ethoxylated fatty alcohols such as lauryl alcohol, laureth-4, laureth-7, laureth-9, laureth-40, trideceth alcohol, C11-15 pareth-9, C12-13 Pareth-3, and C14-15 Pareth-11, alkylpolyglucosides such as decyl glucoside, lauryl glucoside, and coco glucoside.

Zwitterionic surfactants are known. Any Zwitterionic surfactant that is acceptable for use in the intended end use application is suitable as the optional Zwitterionic surfactant component of the composition of the present invention, including, for example, those which can be broadly described as derivatives of aliphatic quaternary ammonium, phosphonium, and sulfonium compounds in which the aliphatic radicals can be straight chain or branched and wherein one of the aliphatic substituents contains from about 8 to 18 carbon atoms and one contains an anionic water-solubilizing group such as carboxyl, sulfonate, sulfate, phosphate or phosphonate. Specific examples of suitable Zwitterionic surfactants include alkyl betaines, such as cocodimethyl carboxymethyl betaine, lauryl dimethyl carboxymethyl betaine, lauryl dimethyl alpha-carboxy-ethyl betaine, cetyl dimethyl carboxymethyl betaine, lauryl bis-(2-hydroxy-ethyl)carboxy methyl betaine, stearyl bis-(2-hydroxypropyl)carboxymethyl betaine, oleyl dimethyl gamma-carboxypropyl betaine, and lauryl bis-(2-hydroxypropyl)alpha-carboxyethyl betaine, amidopropyl betaines, and alkyl sultaines, such as cocodimethyl sulfopropyl betaine, stearyldimethyl sulfopropyl betaine, lauryl dimethyl sulfoethyl betaine, lauryl bis-(2-hydroxy-ethyl)sulfopropyl betaine, and alkylamidopropylhydroxy sultaines.

Amphoteric surfactants are known. Any amphoteric surfactant that is acceptable for use in the intended end use application is suitable as the optional amphoteric surfactant component of the composition of the present invention, including, for example, derivatives of aliphatic secondary and tertiary amines in which the aliphatic radical can be straight chain or branched and wherein one of the aliphatic substituents contains from about 8 to about 18 carbon atoms and one contains an anionic water solubilizing group. Specific examples of suitable amphoteric surfactants include the alkali metal, alkaline earth metal, ammonium or substituted ammonium salts of alkyl amphocarboxy glycinates and alkyl amphocarboxypropionates, alkyl amphodipropionates, alkyl amphodiacetates, alkyl amphoglycinates, and alkyl amphopropionates, as well as alkyl iminopropionates, alkyl iminodipropionates, and alkyl amphopropylsulfonates , such as for example, cocoamphoacetate cocoamphopropionate, cocoamphodiacetate, lauroamphoacetate, lauroamphodiacetate , lauroamphodipropionate, lauroamphodiacetate, cocoamphopropyl sulfonate caproamphodiacetate, caproamphoacetate, caproamphodipropionate, and stearoamphoacetate.

In one embodiment, the structured surfactant composition of the present invention may optionally comprise, based on 100 pbw of the total amount of surfactants present in such structured surfactant composition:
up to about 20 pbw, more typically from about 0.1 to about 10, and still more typically from about 0.5 to about 6, of a cationic surfactant,
up to about 20 pbw, more typically from about 0.5 to 10, and still more typically from about 1 to about 6 of a nonionic surfactant, and
up to about 25 pbw, more typically from about 1 to about 20, and still more typically from about 2 to about 10 of an Zwitterionic or amphoteric surfactant.

In one embodiment, the structured surfactant composition of the present invention comprises, based on 100 pbw of the composition and inclusive of any surfactant used as a structuring agent, a total amount of from about 0.1 to about 40 pbw, more typically from about 0.5 to about 30 pbw, and still more typically from about 1 to about 15 pbw, of one or more cationic surfactants, nonionic surfactants, amphoteric surfactants, and/or zwitterionic surfactants.

Electrolytes suitable as an additional structurant component of the composition of the present invention include salts of multivalent anions, such as potassium pyrophosphate, potassium tripolyphosphate, and sodium or potassium citrate, salts of multivalent cations, including alkaline earth metal salts such as calcium chloride and calcium bromide, as well as zinc halides, barium chloride and calcium nitrate, salts of monovalent cations with monovalent anions, including alkali metal or ammonium halides, such as potassium chloride, sodium chloride, potassium iodide, sodium bromide, and ammonium bromide, alkali metal or ammonium nitrates, and polyelectrolytes, such as uncapped polyacrylates, polymaleates, or polycarboxylates, lignin sulphonates or naphthalene sulphonate formaldehyde copolymers. Electrolytes may be added as a separate component of the structured surfactant or may be added as a part of another component of the composition, e.g., amphoteric surfactants, such as sodium lauroamphoacetate, typically contain an electrolyte, such as sodium chloride.

In one embodiment, the structured surfactant composition, surfactant blend and personal care composition of the present invention each comprise from greater than 0 to about 10 pbw, more typically from about 0.5 to about 6 pbw, still more typically from about 2 to about 4 pbw of electrolyte.

The structured surfactant composition of the present invention may optionally further comprise one or more preservatives, such as benzyl alcohol, methyl paraben, propyl paraben, or imidazolidinyl urea, and DMDM hydantoin, and may optionally further comprise one or more pH adjusting agents, such as citric acid, succinic acid, phosphoric acid, sodium hydroxide, or sodium carbonate.

The structured surfactant composition of the present invention may optionally further comprise one or more polymers and/or thickeners, chosen from the groups of clays, substituted or unsubstituted hydrocolloids, acrylates, acrylates/C10-30 alkyl acrylates crosspolymers, such as, for example, Some examples of clays include bentonite, kaolin, montmorillonite, sodium magnesium silicate, hectorite, magnesium aluminum silicate. Some examples hydrocolloids in the unmodified form include agar, alginate, arabinoxylan, carrageenan, cellulose derivatives, such as carboxyalkyl celluose, hydroxyalkyl cellulose, hydroxyalkyl alkyl cellulose, and alkyl cellulose, curdlan, gelatin, gellan, β-glucan, guar gum, gum arabic, locust bean gum, pectin, starch, succinoglycan, Xanthan gum. Some examples of modified or substituted hydrocolloids are cellulose derivatives, such as carboxyalkyl cellulose, hydroxyalkyl cellulose, hydroxyalkyl alkyl cellulose, alkyl cellulose, hydroxy methyl cellulose, PG-hydroxyethyl cellulose, quaternary ammonium derivatives of hydroxyethyl cellulose, quaternary ammonium derivatives of guar gum (such as Jaguar C-17, Jaguar C-14S, Jaguar Excel, Jaguar C-162 from Rhodia), hydroxypropyl guars (Jaguar HP-8, Jaguar HP-105, Jaguar HP-60, Jaguar HP-120, Jaguar C-162), modified starches, such as sodium hydroxypropyl starch phosphate (Pure-Gel 980 and Pure-Gel 998 from Grain Processing Corporation), potato starch modified (such as Structure-Solanace from National Starch), acrylate copolymers such as acrylates/aminoacrylates/C10-30 alkyl PEG-20 itaconate copolymer (such as Structure-Plus from National Starch), cationic polymers (such as Rheovis CSP, Rheovis CDE, Rheovis CDP from Ciba), polyacrylimidomethylpropane Sulfonate / Polyquaternium-4 (Plexagel ASC from ISP), hydrohobically modified nonionic polyols (Acusol 880, Acusol 882 from Rohm & Haas), and PEG-150 distearate. In general, personal care compositions may optionally comprise, based on 100 pbw of the personal care composition and independently for each such ingredient, up to about 10 pbw, typically from about 0.1 pbw to about 5.0 pbw, more typically from about 0.5 pbw to about 4.0 pbw, of such other ingredients, depending on the desired properties of the personal care composition.

In one embodiment, the structured surfactant composition is made by combining and mixing the anionic surfactant, the amine oxide, and water and, optionally, adjusting the pH. Mixing may be applied as required to form a homogeneous solution.

In one embodiment, the structured surfactant is subjected to a high shear mixing in known mixing equipment, such as, for example, a high shear mixer or a homogenizer.

Shear-thinning viscosity is measured by known viscometric methods, such as for example, using a rotational viscometer, such as a Brookfield viscometer or a cone and plate rheometer. In one embodiment, the composition of the present invention exhibits shear-thinning behavior when subjected to viscosity measurement using a Brookfield rotational viscometer, equipped with an appropriate spindle, at a rotation speed of from about 0.1 revolutions per minute ("rpm") to about 60 rpm.

The structured surfactant composition of the present invention is capable of suspending water-insoluble particles or partially water soluble components, such as vegetable oils, mineral oils, silicone oils, solid particles, abrasives, and similar articles. The composition provides a means to include otherwise difficult to incorporate components in surfactant mixtures resulting in cosmetic preparations with multi-functional benefits including, in some cases, cleansing, moisturizing, improved skin feel, exfoliation/abrasion, novel appearance, or a combination of these benefits.

The ability of a composition to suspend water insoluble or partially water insoluble components is typically evaluated by mixing the composition with sufficient vigor to entrap air bubbles in the composition and then visually observing whether the air bubbles remain entrapped in the composition for a defined period of time, such as for example, 12 to 24 hours, under defined environmental conditions, such as for example, room temperature. In one embodiment, the composition of the present invention is capable of suspending air bubbles for at least 1 week, and more typically for at least 3 months. A composition that is capable of suspending air bubbles under the for at least 12 hours at room temperature is deemed to be generally capable of suspending water insoluble or partially water soluble components in the composition under generally anticipated processing, storage, and use conditions for such composition. For components other than air, the result of the air suspension test should be confirmed by conducting an analogous suspension test using the component of interest. For unusually rigorous processing, storage and/or use conditions, more rigorous testing may be appropriate.

In one embodiment, the ability to suspend water insoluble or partially water insoluble components is evaluated under more rigorous conditions, that is, the mixed samples are visually evaluated after subjecting the samples to one or more freeze/thaw cycles, wherein each freeze/thaw cycle consists of 12 hours at -10°C and 12 hours at 25°C. In one embodiment, composition of the present invention remains capable of suspending air bubbles after one freeze/thaw cycle, more typically after 3 freeze/thaw cycles.

In one embodiment, the structured surfactant composition of the present invention further comprises one or more water insoluble or partially water soluble components. Such components may be in the form of a solid, a liquid, or a gas and may comprise one or more materials selected from water insoluble or partially water soluble benefit agents, such as, for example, in the case of a personal care application, emollients, conditioners, moisturizers, vitamins, vitamin derivatives, moisturizing beads, natural or synthetic abrasives, such as polyoxyethylene beads, anti-UV agents, anti-bacterial agents, anti-fungal agents, tanning accelerators, anti-aging agents, anti-wrinkle agents, antiperspirants, deodorants, essential oils, fragrances, air, or abrasives, and water insoluble or partially water soluble chemically stable appearance modifying additives such as, for example, pigments, opacifying agents, colored or reflective particles or beads such as particles of mica, titanium dioxide, or glycol stearate. It is preferred that the benefit agents are chemically stable in the chosen surfactant system.

In one embodiment, the structured surfactant composition of the present invention is present as a structured surfactant component that forms a first "phase" (which may itself comprise a plurality of phases, including aqueous phases, laminar surfactant phases and spherulitic surfactant phases, as discussed above) of a multi-phase composition that further comprises one or more additional phases that are at least substantially distinct from such first phase. As used herein in reference to the phases of a multiphase embodiments of the present invention, the terminology "substantially distinct" means that the phases each exhibit substantially homogeneous properties within a given phase and that the phases differ with respect to at least one characteristic or property, such as for example, visual characteristics, such as color, clarity, pearlescence, or physical/chemical properties, such as viscosity, lubricity, and/or benefit agent content.

In one embodiment, the structured surfactant component forms a first phase and the composition further comprises at least one additional phase that is at least substantially distinct from the first phase wherein each of such phases is a continuous phase and the phases are disposed adjacent to each other.

In one embodiment, the structured surfactant component forms a first phase and the composition further comprises at least one additional phase that is at least substantially distinct from the first phase wherein one of such phases is a continuous phase, the other of such phases is a discontinuous phase, and the discontinuous phase is dispersed within the continuous phase.

In one embodiment, the structured surfactant component forms a first phase and the composition further comprises at least one additional phase wherein that is at least substantially visually distinct from the first phase, such as for example, a composition comprising an opaque water insoluble component suspended in structured surfactant component.

In one embodiment, the structured surfactant component forms a first phase that exhibits shear-thinning viscosity and is capable of suspending water insoluble or partially water soluble components.

In one embodiment, the structured surfactant component forms a first phase, typically a continuous phase, that exhibits shear-thinning viscosity and is capable of suspending water insoluble or partially water soluble components and the composition further comprises at least one additional phase, typically a discontinuous phase, that is at least substantially distinct form the first phase, wherein the additional phase comprises one or more water insoluble or partially water soluble components.

In another embodiment, the structured surfactant component forms a first phase that exhibits shear-thinning viscosity and is capable of suspending water insoluble or partially water soluble components and the composition further comprises at least one additional phase, such as a second structured surfactant component, that is at least substantially distinct from the first phase and that exhibits shear-thinning viscosity and is capable of suspending water insoluble or partially water soluble components.

In one embodiment, the composition of the present invention comprises two distinct phases, wherein each of the phases is a continuous phase and the phases are disposed adjacent to each other.

In one embodiment, the composition of the present invention comprises two distinct phases, wherein one phase is a continuous phase, the other phase is a discontinuous phase, and the discontinuous phase is adjacent to or dispersed within the continuous phase.

In one embodiment, the composition of the present invention comprises two distinct phases, wherein each phase is a continuous phase and the two phases are disposed in a mutually interpenetrating network.

In one embodiment, a personal care composition of the present invention comprises two or more visually distinct phases. In one embodiment, the two or more visually distinct phases exhibit a visual appearance of alternating stripes.

The composition of the present invention is useful in, for example, personal care applications, such as shampoos, body wash, hand soap, lotions, creams, conditioners, shaving products, facial washes, neutralizing shampoos, personal wipes, and skin treatments, and in home care applications, such as liquid detergents, laundry detergents, hard surface cleansers, dish wash liquids, toilet bowl cleaners, as well as other applications, such as oil field and agrochemical applications.

In one embodiment, the composition of the present invention is a personal care composition.

In one embodiment, the personal care composition of the present invention comprises a structured surfactant composition of the present invention in combination with additional water and/or one or more additional ingredients and suitable personal care compositions are made by diluting the structured surfactant composition with water and/or mixing the structured surfactant composition with additional ingredients.

In one embodiment, the personal care composition consists essentially of the structured surfactant composition of the present invention, i.e., the structured surfactant composition is simply repackaged as a personal care composition.

In one embodiment, the personal care composition of the present invention further comprises one or more benefit agents, such as emollients, moisturizers, conditioners, skin conditioners, or hair conditioners such as vegetable oils, including arachis oil, castor oil, cocoa butter, coconut oil, com oil, cotton seed oil, olive oil, palm kernel oil, rapeseed oil, safflower seed oil, sesame seed oil and soybean oil, esters, including butyl myristate, cetyl palmitate, decyloleate, glyceryl laurate, glyceryl ricinoleate, glyceryl stearate, glyceryl isostearate, hexyl laurate, isobutyl palmitate, isocetyl stearate, isopropyl isostearate, isopropyl laurate, isopropyl linoleate, isopropyl myristate, isopropyl palmitate, isopropyl stearate, propylene glycol monolaurate, propylene glycol ricinoleate, propylene glycol stearate, and propylene glycol isostearate, animal fats, including acetylated lanolin alcohols, lanolin, lard, mink oil and tallow, and fatty acids and alcohols, including behenic acid, palmitic acid, stearic acid, behenyl alcohol, cetyl alcohol, eicosanyl alcohol and isocetyl alcohol; vitamins or their derivatives, such as vitamin B complex, including thiamine, nicotinic acid, biotin, pantothenic acid, choline, riboflavin, vitamin B6, vitamin B12, pyridoxine, inositol, carnitine, vitamins A,C,D,E,K and their derivatives, such as vitamin A palmitate, and pro-vitamins, e.g., panthenol (pro vitamin B5), panthenol triacetate and mixtures thereof; antioxidants; free-radical scavengers; abrasives, natural or synthetic; dyes; hair coloring agents; bleaching agents; hair bleaching agents; UV absorbers, such as benzophenone, bornelone, PABA (Para Amino Benzoic Acid), butyl PABA, cinnamidopropyl trimethyl ammonium chloride, disodium distyrylbiphenyl disulfonate, potassium methoxycinnamate; anti-UV agents, such as butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, aminobenzoic acid, cinoxate, diethanolamine methoxycinnamate, glyceryl aminobenzoate, titanium dioxide, zinc oxide, oxybenzone, octyl dimethyl PABA (padimate O), red petrolatum; antimicrobial agents; antibacterial agents, such as bacitracin, erythromycin, triclosan, neomycin, tetracycline, chlortetracycline, benzethonium chloride, phenol, parachlorometa xylenol (PCMX), triclocarban (TCC), chlorhexidine gluconate (CHG), zinc pyrithione, selenium sulfide; antifungal agents; melanin regulators; tanning accelerators; depigmenting agents, such as retinoids such as retinol, kojic acid and its derivatives such as, for example, kojic dipalmitate, hydroquinone and its derivatives such as arbutin, transexamic acid, vitamins such as niacin, vitamin C and its derivatives, azelaic acid, placertia, licorice, extracts such as chamomile and green tea, where retinol, kojic acid, and hydroquinone are preferred; skin lightening agents such as hydroquinone, catechol and its derivatives, ascorbic acid and its derivatives; skin-coloring agents, such as dihydroxyacetone; liporegulators; weight-reduction agents; anti-acne agents; antiseborrhoeic agents; anti-ageing agents; anti-wrinkle agents; keratolytic agents; anti-inflammatory agents; anti-acne agents, such as tretinoin, isotretinoin, motretinide, adapalene, tazarotene, azelaic acid, retinol, salicylic acid, benzoyl peroxide, resorcinol, antibiotics such as tetracycline and isomers thereof, erythromycin, anti-inflammatory agents such as ibuprofen, naproxen, hetprofen, botanical extracts such as alnus, amica, artemisia capillaris, asiasarum root, calendula, chamomile, cnidium, comfrey, fennel, galla rhois, hawthorn, houttuynia, hypericum, jujube, kiwi, licorice, magnolia, olive, peppermint, philodendron, salvia, sasa albomarginata, imidazoles such as ketoconazole and elubiol, those anti-acne agents described in Gollnick, H. et al. 196(I) Dermatology Sebaceous Glands, Acne and Related Disorders, 119-157 (1998), which is incorporated by reference herein to the extent that it is not inconsistent with the present application; refreshing agents; cicatrizing agents; vascular-protection agents; agents for the reduction of dandruff, seborrheic dermatitis, or psoriasis, such as zinc pyrithione, shale oil and derivatives thereof such as sulfonated shale oil, selenium sulfide, sulfur, salicylic acid, coal tar, povidone-iodine, imidazoles such as ketoconazole, dichlorophenyl imidazolodioxalan, clotrimazole, itraconazole, miconazole, climbazole, tioconazole, sulconazole, butoconazole, fluconazole, miconazolenitrite and any possible stereo isomers and derivatives thereof such as anthralin, piroctone olamine (Octopirox), selenium sulfide, ciclopirox olamine, anti-psoriasis agents such as vitamin D analogs, e.g. calcipotriol, calcitriol, and tacaleitrol, vitamin A analogs such as esters of vitamin A including vitamin A palmitate, retinoids, retinols, and retinoic acid, corticosteroids such as hydrocortisone, clobetasone, butyrate, clobetasol propionate; antiperspirants or deodorants, such as aluminum chlorohydrates, aluminum zirconium chlorohydrates; immunomodulators; nourishing agents; depilating agents, such as calcium thioglycolate, magnesium thioglycolate, potassium thioglycolate, strontium thioglycolate; agents for combating hair loss; reducing agents for permanent-waving; reflectants, such as mica, alumina, calcium silicate, glycol dioleate, glycol distearate, silica, sodium magnesium fluorosilicate; essential oils and fragrances.

In one embodiment, the surfactant composition of the present invention comprises a benefit agent selected from insoluble or partially insoluble ingredients such as moisturizers or conditioners, hair coloring agents, anti-UV agents, anti-wrinkle agents, fragrances or essential oils, skin-coloring agents, anti-dandruff agents, and provides enhanced deposition of such benefit agent on the substrate, ex. hair and/or skin or fabric or counter top or plant leaves.

In one embodiment, the personal care composition of the present invention further comprises from about 0.1 to about 50 pbw, more typically from about 0.3 to about 25 pbw, and still more typically from about 0.5 to 10 pbw, of one or more benefit agents.

The personal care composition according to the present invention may optionally further comprise other ingredients, such as, for example, preservatives such as benzyl alcohol, methyl paraben, propyl paraben and imidazolidinyl urea, thickeners and viscosity modifiers such as block polymers of ethylene oxide and propylene oxide, electrolytes, such as sodium chloride, sodium sulfate, and polyvinyl alcohol, pH adjusting agents such as citric acid, succinic acid, phosphoric acid, sodium hydroxide, and sodium carbonate, perfumes, dyes, and sequestering agents, such as disodium ethylenediamine tetra-acetate. In general, personal care compositions may optionally comprise, based on 100 pbw of the personal care composition and independently for each such ingredient, up to about 10 pbw, preferably from 0.5 pbw to about 5.0 pbw, of such other ingredients, depending on the desired properties of the personal care composition.

In general, personal care composition of the present invention may optionally comprise, based on 100 pbw of the personal care composition and independently for each such ingredient, up to about 15 pbw, preferably from 0.5 pbw to about 10 pbw, of such other ingredients, depending on the desired properties of the personal care composition.

The personal care composition of the present invention is used in a manner know in the art, for example, in the case of a cleanser or shampoo, by application of the cleanser or shampoo to the skin and/or hair and optionally rinsing the cleanser or shampoo off of the skin and/or hair with water.

In one embodiment, the structured surfactant composition, surfactant blend, and personal care composition of the present invention each comprise, based on 100 pbw of such composition, from 0 to less than 20 pbw sugar, typically from 0 to less than 10 pbw, more typically from 0 to less than 5 pbw sugar, still more typically from 0 to less than 3 pbw, and still more typically from 0 to less than 2 pbw, still more typically from 0 to less than 1 pbw, sugar per 100 pbw of the composition. In one embodiment, the structured surfactant composition, surfactant blend, and personal care composition of the present invention each comprise substantially no sugar, i.e., from 0 to less than 0.1 pbw sugar per 100 pbw of the composition, more typically no sugar, i.e., 0 pbw sugar per 100 pbw of the composition. As used herein, the term "sugar" includes monosaccharides, such as glucose and fructose, and disaccharides, such as saccharose, sucrose, lactose, and maltose, as well as mixtures thereof. Sugars are not desirable components of the structured surfactant composition or surfactant blend compositions of the present invention that are to be used in personal care applications, because sugars typically have a detrimental effect on skin feel and lubricity and may undesirably decrease foaming.

### Examples 1 -10

The compositions of Examples 1 - 10 were made by mixing the components to give the relative amounts listed in TABLES I, II and III below (as pbw active ingredient per 100 pbw of composition) Typically, this involved combining ingredients that already contained some water in relative amounts effective to provide the specified level of active ingredient.

The viscosity of each of the compositions of Examples 1-4 was measured using a Brookfield RVF Viscometer equipped with a SP # 3 spindle at 12 revolutions per minute at 25°C for 1 min for each of a series of samples that were subjected to different treatment conditions, that is, an initial viscosity measurement, after storage for 5 days at 45°C, after 5 days of 12 hour freeze (-10)/12 hour thaw (25°C) cycling, and after 5 days storage at 4°C. The viscosity of each of the compositions of Examples 5 and 6 was measured in an analogous manner, but using a Brookfield RVF Viscometer equipped with a T bar B at 2 revolutions per minute. The results are given in the TABLES below in units of centiPoise ("cP").

The stability of each of the compositions was evaluated by visual inspection following the different treatment conditions. Results are given in the TABLES below as "stable" which indicates that the samples had remained as a single phases, and "separated", which indicates that the sample had separated into two phases when centrifuged at 20,000 G for - 15 minutes in 2 milliliter centrifuge tubes.

The ability of the compositions to suspend water insoluble or partially water insoluble components was evaluated by mixing the composition with sufficient vigor to entrap air bubbles in the composition and then visually observing whether the air bubbles remained entrapped in the composition after storing the composition overnight at room temperature.

**TABLE I**

| | **EX.1** | **EX.2** | **EX. 3*** | **EX.4*** |
|---|---|---|---|---|
| **Components (as pbw active ingredient)** | | | | |
| sodium trideceth sulfate | 9.0 | 8.9 | 4.4 | 4.4 |
| sodium lauroamphoaceate | 9.0 | 8.9 | 4.4 | 4.4 |
| water | 77.3 | 77.2 | 86.7 | 86.7 |
| Glydant | 0.4 | 0.4 | 0.2 | 0.2 |
| citric acid | 1.5 | 1.5 | 0.9 | 0.9 |
| sodium chloride | 0.6 | 0.6 | 0.5 | 0.5 |
| lauramine oxide | 2.2 | 2.5 | 2.5 | 2.5 |
| xanthan gum (Rhodicare T) | - | - | 0.4 | - |
| guar qum (Jaquar S) | - | - | - | 0.4 |

| **Viscosity** (cP), Brookfield LVT Viscometer, SP # 3,12 RPM, 25°C, 1 min | | | | |
|---|---|---|---|---|
| - Initial | 2070 | 2200 | 2530 | 2800 |
| - 5 Days at 45°C | 2070 | 2130 | 2470 | 2660 |
| - 5 Days Freeze/Thaw Cycle (-10/25°C) | 2130 | 2400 | 2630 | 3270 |
| - 5 Days at 4°C) | 2080 | 2240 | 2960 | 2720 |
| **Suspends Air?** | Yes | Yes | Yes | Yes |

**TABLE II**

| | **EX.5** | **EX.6** |
|---|---|---|
| **Components (as pbw active ingredient)** | | |
| Water | 60.2 | 60.9 |
| guar hydroxypropyltrimonium chloride (Jaguar C-17) | 0.4 | 0.4 |
| guar gum (Jaguar S) | 0.4 | 0.4 |
| lauramine oxide | 2.9 | 2.5 |
| sodium lauroamphoacetate | 0.4 | 0.4 |
| sodium laureth sulfate | 7.4 | 7.4 |
| petrolatum | 25.2 | 25.0 |
| citric acid | 0.6 | 0.6 |
| Glydant | 0.4 | 0.4 |
| sodium chloride | 2.1 | 2.0 |

| **Viscosity (cP)** | | |
|---|---|---|
| - Initial | 35600 | 35800 |
| - 5 Days 45°C | 33000 | 32200 |
| - 5 Days Freeze/Thaw Cycles (-10/25°C) | 32000 | 30400 |
| - 5 Days at 4°C | 40800 | 37000 |
| **Suspends Air?** | Yes | Yes |

**TABLE III**

| | **EX. 7*** | **EX. 8** | **Ex. 9** | **Ex. 10** |
|---|---|---|---|---|
| **Components** (as pbw active ingredient) | | | | |
| water | 79.2 | 79.9 | 79.9 | 79.9 |
| sodium trideceth sulfate | 14.7 | 14.7 | 14.7 | 14.7 |
| sodium chloride | 4.0 | 4.0 | 4.0 | 4.0 |
| citric acid | 0.7 | 0.0 | 0.0 | 0.0 |
| cocamide MEA | -- | 1.3 | -- | -- |
| laureth-2 | -- | -- | 1.3 | -- |
| sodium chloride | -- | -- | -- | 1.3 |
| lauramine oxide | 1.3 | -- | -- | -- |
| **Stability** | stable | separated | separated | separated |
| **Suspends Air?** | Yes | No | No | No |

| | | | | |
|---|---|---|---|---|
| *not according to the invention | | | | |

## Claims

1. An aqueous structured surfactant composition, comprising based on 100 parts by weight of the composition:
water,
from greater than 0 to 6 parts by weight of an electrolyte selected from potassium pyrophosphate, potassium tripolyphosphate, sodium citrate, potassium citrate, calcium chloride, calcium bromide, zinc halides, barium chloride, calcium nitrate, alkali metal halides, ammonium halides, alkali metal nitrate, ammonium nitrates, uncapped polyacrylates, polymaleates, polycarboxylates, lignin sulphonates and naphthalene sulphonate formaldehyde copolymers,
from 8 to 40 parts by weight of one or more anionic surfactants selected from linear alkylbenzene sulfonates, alpha olefin sulfonates, paraffin sulfonates, alkyl ester sulfonates, alkyl sulfates, alkyl alkoxy sulfates, alkyl sulfonates, alkyl alkoxy carboxylates, alkyl alkoxylated sulfates, monoalkyl phosphates, dialkyl phosphates, sarcosinates, sulfosuccinates, isethionates, and taurates, and mixtures thereof,
and greater than 0 to 5 parts by weight of one or more amine oxides according to formula (III): 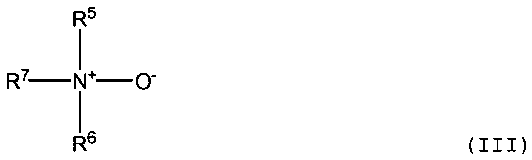
wherein R⁵, R⁶, and R⁷ are each independently alkyl, hydroxyalkyl, alkoxyl, alkenyl, R⁸-R⁹- R¹⁰-C(O-NH-R¹¹-, a phosphate moiety, a phosphonate moiety, or any two of R⁵, R⁶, and R⁷ are fused to form a 5- to 8-membered saturated or unsaturated heterocyclic ring that includes the nitrogen atom of the amine oxide and, optionally, further includes a second nitrogen atom or an oxygen atom as a ring member and which may be further substituted with alkyl or amino on one or more of the ring atoms,
R⁸ and R¹⁰ are each independently H, alkyl, or alkenyl,
R⁹ is alkyleneoxy, and
R¹¹ is alkylene or alkyleneoxy,
wherein the amount of amine oxide is from 10 to 50 parts by weight per 100 parts by weight of the anionic surfactant, said composition exhibiting shear-thinning viscosity and is capable of suspending water insoluble or partially water soluble components.

2. The composition of claim 1, wherein the composition comprises, based on 100 parts by weight of the composition, from 1.5 to 5 parts by weight of the one or more amine oxides, preferably 2 to 5 parts by weight of the one or more amine oxides.

3. The composition of claim 1, wherein R⁵, R⁶, and R⁷ are each independently (C₁-C₅₀)alkyl, (C₁-C₅₀)hydroxyalkyl, (C₁-C₅₀)alkoxyl, (C₂-C₅₀) alkenyl, R⁸-R⁹ , R¹⁰-C(O)-NH-R¹¹, a phosphate moiety, a phosphonate moiety, or any two of R⁵, R⁶, and R⁷ are fused to form a 5- or 6-membered saturated or unsaturated heterocyclic ring that includes the nitrogen atom of the amine oxide and, optionally, further includes a second nitrogen atom or an oxygen atom as a ring member and which may be further substituted with (C₁-C₅₀)alkyl or amino on one or more of the ring atoms,
R⁸ and R¹⁰ are each independently H, (C₁-C₅₀)alkyl, or (C₂-C₅₀)alkenyl,
R⁹ is (C₁-C₅₀)alkyleneoxy, and
R^{11 is} (C₁-C₅₀)alkylene or (C₂-C₅₀)alkyleneoxy.

4. The composition of claim 1, wherein at least one of R⁵, R⁶, and R⁷ is (C₁-C₅₀)alkyl, (C₁-C₅₀)hydroxyalkyl, (C₁-C₅₀)alkoxyl, or (C₂-C₅₀)alkenyl.

5. The composition of claim 1, wherein at least one of R⁵, R⁶, and R⁷ is derived from almond oil, babassu oil, hydrogenated palm kernel oil, hydrogenated tallow, milk, mink oil, olive oil, palm oil, sesame oil, soy, tallow, or wheat germ oil.

6. The composition of claim 1, wherein one of R⁵, R⁶, and R⁷ is (C₈-C₅₀)alkyl or (C₈-C₅₀)alkenyl and the other two of R⁵, R⁶, and R⁷ are each independently (C₁-C₇) alkyl, (C₁-C₇) hydroxyalkyl, (C₁-C₇)alkoxyl.

7. The composition of claim 1, wherein at least one of R⁵, R⁶, and R⁷ is R¹⁰_C(O) -NH-^{R}11 , wherein R¹⁰ is (C₁-C₅₀)alkyl or (C₂-C₅₀) alkenyl, and R¹¹ is (C₁-C₂₀)alkylene, typically methylene or (C₂-C₂₀) polymethylene.

8. The composition of claim 1, wherein at least one of R⁵, R⁶, and R⁷ is a radical R¹⁰-C(O)-NH-R¹¹, wherein R¹⁰ is (C₁-C₅₀)alkyl or (C₂-C₅₀)alkenyl and the R¹⁰-C(O)- portion of the radical is derived from almond oil, babassu oil, hydrogenated palm kernel oil, hydrogenated tallow, milk, mink oil, olive oil, palm oil, sesame oil, soy, tallow, or wheat germ oil, and R¹¹ is (C₁-C₂₀)alkylene, typically methylene or (C₂-C₂₀)polymethylene.

9. The composition of claim 1, wherein two of R⁵, R⁶, and R⁷ are fused to form a 5- or 6-membered saturated or unsaturated heterocyclic ring which includes the nitrogen atom of the amine oxide and a second nitrogen atom as ring members and wherein the ring is substituted with amino on one or more of the ring atoms.

10. The composition of claim 1, wherein two of R⁵, R⁶, and R⁷ are fused to form a 5- or 6-membered saturated or unsaturated heterocyclic ring which includes the nitrogen atom of the amine oxide and an oxygen atom as ring members and wherein the ring is substituted with alkyl on one or more of the ring carbons.

11. The composition of claim 1, wherein the composition further comprises from 0.5 to 30 parts by weight of one or more surfactants selected from cationic surfactants, nonionic surfactants, amphoteric surfactants, and zwitterionic surfactants.

12. A method for making an aqueous structured surfactant composition, comprising mixing, based on 100 parts by weight of the composition:
water,
from greater than 0 to 6 parts by weight of an electrolyte selected from potassium pyrophosphate, potassium tripolyphosphate, sodium citrate, potassium citrate, calcium chloride, calcium bromide, zinc halides, barium chloride, calcium nitrate, alkali metal halides, ammonium halides, alkali metal nitrate, ammonium nitrates, uncapped polyacrylates, polymaleates, polycarboxylates, lignin sulphonates and naphthalene sulphonate formaldehyde copolymers,
from 8 to 40 parts by weight of one or more anionic surfactants selected from linear alkylbenzene sulfonates, alpha olefin sulfonates, paraffin sulfonates, alkyl ester sulfonates, alkyl sulfates, alkyl alkoxy sulfates, alkyl sulfonates, alkyl alkoxy carboxylates, alkyl alkoxylated sulfates, monoalkyl phosphates, dialkyl phosphates, sarcosinates, sulfosuccinates, isethionates, and taurates, and mixtures thereof,
and greater than 0 to 5 parts by weight of one or more amine oxides according to formula (III): 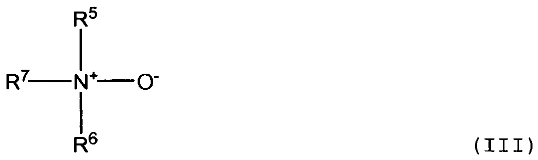
wherein R⁵, R⁶, and R⁷ are each independently alkyl, hydroxyalkyl, alkoxyl, alkenyl, R⁸-R⁹ , R¹⁰-C(O)-NH-R¹¹-, a phosphate moiety, a phosphonate moiety, or any two of R⁵, R⁶, and R⁷ are fused to form a 5- to 8-membered saturated or unsaturated heterocyclic ring that includes the nitrogen atom of the amine oxide and, optionally, further includes a second nitrogen atom or an oxygen atom as a ring member and which may be further substituted with alkyl or amino on one or more of the ring atoms,
R⁸ and R¹⁰ are each independently H, alkyl, or alkenyl,
R⁹ is alkyleneoxy, and
R¹¹ is alkylene or alkyleneoxy,
wherein the amount of amine oxide is from 10 to 50 parts by weight per 100 parts by weight of the anionic surfactant,
wherein the composition exhibits shear-thinning viscosity and is capable of suspending water insoluble or partially water soluble components.

13. A multi-phase composition comprising:
a first phase that comprises, based on 100 parts by weight of the composition:
water,
from greater than 0 to 6 parts by weight of an electrolyte selected from potassium pyrophosphate, potassium tripolyphosphate, sodium citrate, potassium citrate, calcium chloride, calcium bromide, zinc halides, barium chloride, calcium nitrate, alkali metal halides, ammonium halides, alkali metal nitrate, ammonium nitrates, uncapped polyacrylates, polymaleates, polycarboxylates, lignin sulphonates and naphthalene sulphonate formaldehyde copolymers,
from 8 to 40 parts by weight of one or more anionic surfactants selected from linear alkylbenzene sulfonates, alpha olefin sulfonates, paraffin sulfonates, alkyl ester sulfonates, alkyl sulfates, alkyl alkoxy sulfates, alkyl sulfonates, alkyl alkoxy carboxylates, alkyl alkoxylated sulfates, monoalkyl phosphates, dialkyl phosphates, sarcosinates, sulfosuccinates, isethionates, and taurates, and mixtures thereof,
and greater than 0 to 5 parts by weight of one or more amine oxides according to formula (III): 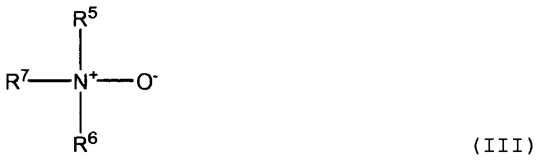 wherein R⁵, R⁶, and R⁷ are each independently alkyl, hydroxyalkyl, alkoxyl, alkenyl, R⁸-R⁹-, R¹⁰-C(O)-NH-R¹¹-, a phosphate moiety, a phosphonate moiety, or any two of R⁵, R⁶, and R⁷ are fused to form a 5- to 8-membered saturated or unsaturated heterocyclic ring that includes the nitrogen atom of the amine oxide and, optionally, further includes a second nitrogen atom or an oxygen atom as a ring member and which may be further substituted with alkyl or amino on one or more of the ring atoms,
R⁸ and R¹⁰ are each independently H, alkyl, or alkenyl,
R⁹ is alkyleneoxy, and
R¹¹ is alkylene or alkyleneoxy,
wherein the amount of amine oxide is from 10 to 50 parts by weight per 100 parts by weight of the anionic surfactant, which exhibits shear-thinning viscosity, and is capable of suspending water insoluble or partially water soluble components, and
one or more additional phases that comprise one or more water insoluble or partially water insoluble components and is at least substantially distinct from the first phase.

14. The composition of claim 13, wherein the composition is a personal care composition and the one or more water insoluble or partially water insoluble components comprise one or more benefit agents.

15. The composition of claim 14, wherein the composition is selected from shampoos, body washes, hand soaps, lotions, creams, conditioners, shaving products, facial washes, personal wipes, and skin treatments.

16. The composition of claim 14, wherein the composition further comprises one or more benefit agents comprising one or more compounds selected from emollients, moisturizers, conditioners, skin conditioners, hair conditioners, vitamins, pro-vitamins, antioxidants, free-radical scavengers, abrasives, dyes, hair coloring agents, bleaching agents, UV absorbers, anti-UV agents, antibacterial agents, antifungal agents, melanin regulators, tanning accelerators, depigmenting agents, skin lightening agents, skin-coloring agents, liporegulators, weight-reduction agents, anti-acne agents, antiseborrhoeic agents, anti-ageing agents, anti-wrinkle agents, keratolytic agents, anti-inflammatory agents, anti-acne agents, antibiotics, anti-inflammatory agents, botanical extracts, imidazoles, refreshing agents, cicatrizing agents, vascular-protection agents, agents for the reduction of dandruff, seborrheic dermatitis, or psoriasis, shale oil and derivatives thereof, anti-psoriasis agents, corticosteroids depilating agents, agents for combating hair loss, reducing agents for permanent-waving, reflectants, essential oils and fragrances.

## Patentansprüche

1. Wässrige strukturierte Tensidzusammensetzung, die - bezogen auf 100 Gewichtsteile der Zusammensetzung - Folgendes umfasst:
Wasser,
mehr als 0 bis 6 Gewichtsteile eines Elektrolyten, der aus Kaliumpyrophosphat, Kaliumtripolyphosphat, Natriumcitrat, Kaliumcitrat, Calciumchlorid, Calciumbromid, Zinkhalogeniden, Bariumchlorid, Calciumnitrat, Alkalimetallhalogeniden, Ammoniumhalogeniden, Alkalimetallnitrat, Ammoniumnitraten, nicht überkappten Polyacrylaten, Polymaleaten, Polycarboxylaten, Ligninsulfonaten und Naphthalinsulfonat-Formaldehyd-Copolymeren ausgewählt ist,
8 bis 40 Gewichtsteile eines oder mehrerer anionischer Tenside, die aus linearen Alkylbenzolsulfonaten, alpha-Olefinsulfonaten, Paraffinsulfonaten, Alkylestersulfonaten, Alkylsulfaten, Alkylalkoxysulfaten, Alkylsulfonaten, Alkylalkoxycarboxylaten, alkylalkoxylierten Sulfaten, Monoalkylphosphaten, Dialkylphosphaten, Sarcosinaten, Sulfosuccinaten, Isethionaten und Tauraten und Gemischen davon ausgewählt sind,
und mehr als 0 bis 5 Gewichtsteile eines oder mehrerer Aminoxide gemäß der Formel (III): 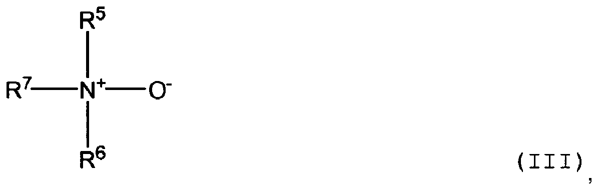
wobei R⁵, R⁶ und R⁷ jeweils unabhängig Alkyl, Hydroxyalkyl, Alkoxyl, Alkenyl, R⁸-R⁹-, R¹⁰-C(O)-NH-R¹¹-, ein Phosphatteil oder ein Phosphonatteil sind oder beliebige zwei von R⁵, R⁶ und R⁷ kondensiert werden, um einen 5- bis 8-gliedrigen gesättigten oder ungesättigten heterocyclischen Ring zu bilden, der das Stickstoffatom des Aminoxids enthält und gegebenenfalls weiterhin ein zweites Stickstoffatom oder ein Sauerstoffatom als ein Ringelement enthält und der weiterhin an einem oder mehreren der Ringatome mit Alkyl oder Amino substituiert sein kann,
R⁸ und R¹⁰ jeweils unabhängig H, Alkyl oder Alkenyl sind,
R⁹ Alkylenoxy ist und
R¹¹ Alkylen oder Alkylenoxy ist,
wobei die Aminoxidmenge 10 bis 50 Gewichtsteile pro 100 Gewichtsteile des anionischen Tensids beträgt, wobei die Zusammensetzung Strukturviskosität zeigt und wasserunlösliche oder teilweise wasserlösliche Bestandteile suspendieren kann.

2. Zusammensetzung nach Anspruch 1, wobei die Zusammensetzung - bezogen auf 100 Gewichtsteile der Zusammensetzung - 1,5 bis 5 Gewichtsteile des einen oder der mehreren Aminoxide, vorzugsweise 2 bis 5 Gewichtsteile des einen oder der mehreren Aminoxide umfasst.

3. Zusammensetzung nach Anspruch 1, wobei R⁵, R⁶ und R⁷ jeweils unabhängig (C₁-C₅₀)-Alkyl, (C₁-C₅₀)-Hydroxyalkyl, (C₁-C₅₀)-Alkoxyl, (C₂-C₅₀)-Alkenyl, R⁸-R⁹-, R¹⁰-C(O)-NH-R¹¹, ein Phosphatteil oder ein Phosphonatteil sind oder beliebige zwei von R⁵, R⁶ und R⁷ kondensiert werden, um einen 5- oder 6-gliedrigen gesättigten oder ungesättigten heterocyclischen Ring zu bilden, der das Stickstoffatom des Aminoxids enthält und gegebenenfalls weiterhin ein zweites Stickstoffatom oder ein Sauerstoffatom als ein Ringelement enthält und der weiterhin an einem oder mehreren der Ringatome mit (C₁-C₅₀)-Alkyl oder Amino substituiert sein kann,
R⁸ und R¹⁰ jeweils unabhängig H, (C₁-C₅₀)-Alkyl oder (C₂-C₅₀)-Alkenyl sind,
R⁹ (C₁-C₅₀)-Alkylenoxy ist und
R¹¹ (C₁-C₅₀)-Alkylen oder (C₂-C₅₀)-Alkylenoxy ist.

4. Zusammensetzung nach Anspruch 1, wobei mindestens einer von R⁵, R⁶ und R⁷ (C₁-C₅₀)-Alkyl, (C₁-C₅₀)-Hydroxyalkyl, (C₁-C₅₀)-Alkoxyl oder (C₂-C₅₀)-Alkenyl ist.

5. Zusammensetzung nach Anspruch 1, wobei mindestens einer von R⁵, R⁶ und R⁷ von Mandelöl, Babassuöl, gehärtetem Palmkernöl, gehärtetem Talg, Milch, Nerzöl, Olivenöl, Palmöl, Sesamöl, Soja-, Talg- oder Weizenkeimöl abgeleitet ist.

6. Zusammensetzung nach Anspruch 1, wobei einer von R⁵, R⁶ und R⁷ (C₈-C₅₀)-Alkyl oder (C₈-C₅₀)-Alkenyl ist und die anderen zwei von R⁵, R⁶ und R⁷ jeweils unabhängig (C₁-C₇)-Alkyl, (C₁-C₇)-Hydroxyalkyl, (C₁-C₇)-Alkoxyl sind.

7. Zusammensetzung nach Anspruch 1, wobei mindestens einer von R⁵, R⁶ und R⁷ R¹⁰_C(O) -NH-R¹¹- ist, wobei R¹⁰ (C₁-C₅₀)-Alkyl oder (C₂-C₅₀)-Alkenyl ist und R¹¹ (C₁-C₂₀)-Alkylen, in der Regel Methylen oder (C₂-C₂₀)-Polymethylen ist.

8. Zusammensetzung nach Anspruch 1, wobei mindestens einer von R⁵, R⁶ und R⁷ ein Rest R¹⁰-C(O)-NH-R¹¹- ist, wobei R¹⁰ (C₁-C₅₀)-Alkyl oder (C₂-C₅₀)-Alkenyl ist und der R¹⁰-C(O)- -Teil des Rests von Mandelöl, Babassuöl, gehärtetem Palmkernöl, gehärtetem Talg, Milch, Nerzöl, Olivenöl, Palmöl, Sesamöl, Soja-, Talg- oder Weizenkeimöl abgeleitet ist und R¹¹ (C₁-C₂₀)-Alkylen, in der Regel Methylen oder (C₂-C₂₀)-Polymethylen ist.

9. Zusammensetzung nach Anspruch 1, wobei zwei von R⁵, R⁶ und R⁷ kondensiert werden, um einen 5- oder 6-gliedrigen gesättigten oder ungesättigten heterocyclischen Ring zu bilden, der das Stickstoffatom des Aminoxids und ein zweites Stickstoffatom als Ringelemente enthält, und wobei der Ring an einem oder mehreren der Ringatome mit Amino substituiert ist.

10. Zusammensetzung nach Anspruch 1, wobei zwei von R⁵, R⁶ und R⁷ kondensiert werden, um einen 5- oder 6-gliedrigen gesättigten oder ungesättigten heterocyclischen Ring zu bilden, der das Stickstoffatom des Aminoxids und ein Sauerstoffatom als Ringelemente enthält, und wobei der Ring an einem oder mehreren der Ringatome mit Alkyl substituiert ist.

11. Zusammensetzung nach Anspruch 1, wobei die Zusammensetzung weiterhin 0,5 bis 30 Gewichtsteile eines oder mehrerer Tenside umfasst, die aus kationischen Tensiden, nichtionischen Tensiden, amphoteren Tensiden und zwitterionischen Tensiden ausgewählt sind.

12. Verfahren zur Herstellung einer wässrigen strukturierten Tensidzusammensetzung, das das Mischen der folgenden Bestandteile - bezogen auf 100 Gewichtsteile der Zusammensetzung - umfasst:
Wasser,
mehr als 0 bis 6 Gewichtsteilen eines Elektrolyten, der aus Kaliumpyrophosphat, Kaliumtripolyphosphat, Natriumcitrat, Kaliumcitrat, Calciumchlorid, Calciumbromid, Zinkhalogeniden, Bariumchlorid, Calciumnitrat, Alkalimetallhalogeniden, Ammoniumhalogeniden, Alkalimetallnitrat, Ammoniumnitraten, nicht überkappten Polyacrylaten, Polymaleaten, Polycarboxylaten, Ligninsulfonaten und Naphthalinsulfonat-Formaldehyd-Copolymeren ausgewählt ist,
8 bis 40 Gewichtsteilen eines oder mehrerer anionischer Tenside, die aus linearen Alkylbenzolsulfonaten, alpha-Olefinsulfonaten, Paraffinsulfonaten, Alkylestersulfonaten, Alkylsulfaten, Alkylalkoxysulfaten, Alkylsulfonaten, Alkylalkoxycarboxylaten, alkylalkoxylierten Sulfaten, Monoalkylphosphaten, Dialkylphosphaten, Sarcosinaten, Sulfosuccinaten, Isethionaten und Tauraten und Gemischen davon ausgewählt sind,
und mehr als 0 bis 5 Gewichtsteilen eines oder mehrerer Aminoxide gemäß der Formel (III): 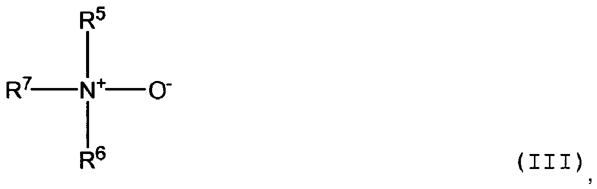
wobei R⁵, R⁶ und R⁷ jeweils unabhängig Alkyl, Hydroxyalkyl, Alkoxyl, Alkenyl, R⁸₋R⁹-, R¹⁰-C(O)-NH-R¹¹-, ein Phosphatteil oder ein Phosphonatteil sind oder beliebige zwei von R⁵, R⁶ und R⁷ kondensiert werden, um einen 5- bis 8-gliedrigen gesättigten oder ungesättigten heterocyclischen Ring zu bilden, der das Stickstoffatom des Aminoxids enthält und gegebenenfalls weiterhin ein zweites Stickstoffatom oder ein Sauerstoffatom als ein Ringelement enthält und der weiterhin an einem oder mehreren der Ringatome mit Alkyl oder Amino substituiert sein kann,
R⁸ und R¹⁰ jeweils unabhängig H, Alkyl oder Alkenyl sind,
R⁹ Alkylenoxy ist und
R¹¹ Alkylen oder Alkylenoxy ist,
wobei die Aminoxidmenge 10 bis 50 Gewichtsteile pro 100 Gewichtsteile des anionischen Tensids beträgt,
wobei die Zusammensetzung Strukturviskosität zeigt und wasserunlösliche oder teilweise wasserlösliche Bestandteile suspendieren kann.

13. Mehrphasige Zusammensetzung, die Folgendes umfasst:
eine erste Phase, die - bezogen auf 100 Gewichtsteile der Zusammensetzung - Folgendes umfasst:
Wasser,
mehr als 0 bis 6 Gewichtsteile eines Elektrolyten, der aus Kaliumpyrophosphat, Kaliumtripolyphosphat, Natriumcitrat, Kaliumcitrat, Calciumchlorid, Calciumbromid, Zinkhalogeniden, Bariumchlorid, Calciumnitrat, Alkalimetallhalogeniden, Ammoniumhalogeniden, Alkalimetallnitrat, Ammoniumnitraten, nicht überkappten Polyacrylaten, Polymaleaten, Polycarboxylaten, Ligninsulfonaten und Naphthalinsulfonat-Formaldehyd-Copolymeren ausgewählt ist,
8 bis 40 Gewichtsteile eines oder mehrerer anionischer Tenside, die aus linearen Alkylbenzolsulfonaten, alpha-Olefinsulfonaten, Paraffinsulfonaten, Alkylestersulfonaten, Alkylsulfaten, Alkylalkoxysulfaten, Alkylsulfonaten, Alkylalkoxycarboxylaten, alkylalkoxylierten Sulfaten, Monoalkylphosphaten, Dialkylphosphaten, Sarcosinaten, Sulfosuccinaten, Isethionaten und Tauraten und Gemischen davon ausgewählt sind,
und mehr als 0 bis 5 Gewichtsteile eines oder mehrerer Aminoxide gemäß der Formel (III): 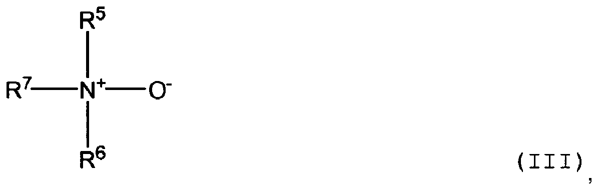
wobei R⁵, R⁶ und R⁷ jeweils unabhängig Alkyl, Hydroxyalkyl, Alkoxyl, Alkenyl, R⁸-R⁹-, R¹⁰-C(O)-NH-R¹¹-, ein Phosphatteil oder ein Phosphonatteil sind oder beliebige zwei von R⁵, R⁶ und R⁷ kondensiert werden, um einen 5- bis 8-gliedrigen gesättigten oder ungesättigten heterocyclischen Ring zu bilden, der das Stickstoffatom des Aminoxids enthält und gegebenenfalls weiterhin ein zweites Stickstoffatom oder ein Sauerstoffatom als ein Ringelement enthält und der weiterhin an einem oder mehreren der Ringatome mit Alkyl oder Amino substituiert sein kann,
R⁸ und R¹⁰ jeweils unabhängig H, Alkyl oder Alkenyl sind,
R⁹ Alkylenoxy ist und
R¹¹ Alkylen oder Alkylenoxy ist,
wobei die Aminoxidmenge 10 bis 50 Gewichtsteile pro 100 Gewichtsteile des anionischen Tensids beträgt, wobei die erste Phase Strukturviskosität zeigt und
wasserunlösliche oder teilweise wasserlösliche Bestandteile suspendieren kann, und
eine oder mehrere zusätzliche Phasen, die eine oder mehrere wasserunlösliche oder teilweise wasserlösliche Bestandteile umfassen und sich von der ersten Phase zumindest im Wesentlichen unterscheiden.

14. Zusammensetzung nach Anspruch 13, wobei die Zusammensetzung eine Körperpflegezusammensetzung ist und die einen oder die mehreren wasserunlöslichen oder teilweise wasserlöslichen Bestandteile ein oder mehrere Pflegemittel umfassen.

15. Zusammensetzung nach Anspruch 14, wobei die Zusammensetzung aus Shampoos, Duschgelen, Handseifen, Lotionen, Cremes, Spülungen, Rasierprodukten, Gesichtsreinigern, Hautpflegetüchern und Hautbehandlungsprodukten ausgewählt ist.

16. Zusammensetzung nach Anspruch 14, wobei die Zusammensetzung weiterhin ein oder mehrere Pflegemittel umfasst, die eine oder mehrere Verbindungen umfassen, die aus Pflegecremes, Feuchtigkeitscremes, Spülungen, Hautpflegecremes, Haarspülungen, Vitaminen, Provitaminen, Antioxidationsmitteln, Radikalfängern, Peelings, Tönungen, Haarfärbemitteln, Bleichmitteln, UV-Absorptionsmitteln, UV-Schutzmitteln, antibakteriellen Mitteln, Antimykotika, melaninregulierenden Mitteln, Bräunungsbeschleunigern, Depigmentierungsmitteln, Hautbleichmitteln, Hautfärbemitteln, Lipidsenkern, Schlankheitsmitteln, Aknemitteln, Antischuppenmitteln, Anti-Aging-Mitteln, Faltenglättern, Keratolytika, entzündungshemmenden Mitteln, Aknemitteln, Antibiotika, entzündungshemmenden Mitteln, Pflanzenextrakten, Imidazolen, Erfrischungsmitteln, die Narbenbildung fördernden Mitteln, Gefäßschutzmitteln, Mitteln zur Verringerung von Schuppen, Seborrhoe oder Schuppenflechte, Schieferöl und Derivaten davon, Mitteln gegen Schuppenflechte, Kortikosteroiden, Enthaarungsmitteln, Mitteln zur Bekämpfung von Haarausfall, Dauerwellmitteln, Reflexionsmitteln, ätherischen Ölen und Duftstoffen ausgewählt sind.

## Revendications

1. Composition de tensio-actif structuré aqueux comprenant, sur la base de 100 parties en poids de la composition :
de l'eau,
de plus de 0 à 6 parties en poids d'un électrolyte sélectionné parmi les pyrophosphate de potassium, tripolyphosphate de potassium, citrate de sodium, citrate de potassium, chlorure de calcium, bromure de calcium, halogénures de zinc, chlorure de baryum, nitrate de calcium, halogénures métalliques alcalins, halogénures d'ammonium, nitrate métallique alcalin, nitrates d'ammonium, polyacrylates non coiffés, polymaléates, polycarboxylates, lignosulphonates et copolymères de sulfonate de naphtalène et de formaldéhyde,
de 8 à 40 parties en poids d'un ou plusieurs tensio-actifs anioniques sélectionnés parmi les sulfonates d'alkyl benzène linéaires, sulfonates d'alpha-oléfine, sulfonates de paraffine, sulfonates d'esters alkyliques, sulfates d'alkyle, sulfates d'alkyle à liaison alkoxy, sulfonates d'alkyle, sulfates d'alkoxy à liaison alkyle, sulfates alcoxylés d'alkyle, phosphates monoalkyles, phosphates dialkyles, sarcosinates, sulfosuccinates, iséthionates et taurates, et leurs mélanges,
et de plus de 0 à 5 parties en poids d'un ou plusieurs oxydes d'amine selon la formule (III) : 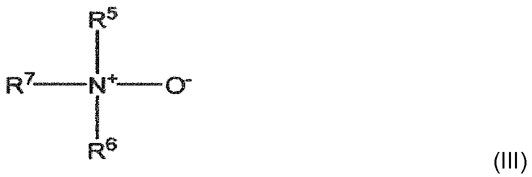
dans laquelle R⁵, R⁶ et R⁷ sont indépendamment les uns des autres alkyle, hydroxyalkyle, alkoxyle, alcényle, R⁸-R⁹-, R¹⁰-C(O)-NH-R¹¹-, une fraction phosphate, une fraction phosphonate, ou deux parmi R⁵, R⁶ et R⁷ sont fusionnés pour former un cycle hétérocyclique saturé ou non saturé de 5 à 8 chaînons qui comprend l'atome azote de l'oxyde d'amine et qui en outre comprend facultativement un deuxième atome azote ou un atome oxygène comme chaînon du cycle, et qui peut être en outre substitué par alkyle ou amino sur un ou plusieurs des atomes du cycle,
R⁸ et R¹⁰ sont indépendamment les uns des autres H, alkyle ou alcényle,
R⁹ est alkylèneoxy, et
R¹¹ est alkylène ou alkylèneoxy,
dans laquelle la quantité d'oxyde d'amine est de 10 à 50 parties en poids pour 100 parties en poids du tensio-actif anionique, ladite composition présentant une viscosité rhéofluidifiante et pouvant mettre en suspension des composants insolubles ou partiellement solubles dans l'eau.

2. Composition selon la revendication 1, dans laquelle la composition comprend, sur la base de 100 parties en poids de la composition, entre 1,5 et 5 parties en poids d'une ou plusieurs oxydes d'amine, de préférence entre 2 et 5 parties en poids de la ou des oxydes d'amine.

3. Composition selon la revendication 1, dans laquelle R⁵, R⁶ et R⁷ sont indépendamment les uns des autres alkyle (C₁-C₅₀), hydroxyalkyle (C₁-C₅₀), alkoxyle (C₁-C₅₀), alcényle (C₂-C₅₀), R⁸-R⁹-, R¹⁰-C(O)-NH-R¹¹-, une fraction phosphate, une fraction phosphonate, ou deux parmi R⁵, R⁶ et R⁷ sont fusionnés pour former un cycle hétérocyclique saturé ou non saturé de 5 à 6 chaînons qui comprend l'atome azote de l'oxyde d'amine et, qui en outre comprend facultativement un deuxième atome azote ou un atome oxygène comme chaînon du cycle, et qui peut être en outre substitué par alkyle (C₁-C₅₀) ou amino sur un ou plusieurs des atomes du cycle,
R⁸ et R¹⁰ sont indépendamment les uns des autres H, alkyle (C₁-C₅₀) ou alcényle (C₂-C₅₀),
R⁹ est alkylèneoxy (C₁-C₅₀), et
R¹¹ est alkylène (C₁-C₅₀) ou alkylèneoxy (C₂-C₅₀).

4. Composition selon la revendication 1, dans laquelle au moins un parmi R⁵, R⁶ et R⁷ est alkyle (C₁-C₅₀), hydroxyalkyle (C₁-C₅₀), alkoxyle (C₁-C₅₀) ou alcényle (C₂-C₅₀).

5. Composition selon la revendication 1, dans laquelle au moins un parmi R⁵, R⁶ et R⁷ est dérivé de : huile d'amande, huile de babassu, huile de noyaux de palme, suif hydrogéné, lait, huile de vison, huile d'olive, huile de palme, huile de sésame, soja, suif ou huile de germe de blé.

6. Composition selon la revendication 1, dans laquelle un parmi R⁵, R⁶ et R⁷ est alkyle (C₈-C₅₀) ou alcényle (C₈-C₅₀) et les deux autres parmi R⁵, R⁶ et R⁷sont indépendamment les uns des autres alkyle (C₁-C₇), hydroxyalkyle (C₁-C₇), alkoxyle (C₁-C₇).

7. Composition selon la revendication 1, dans laquelle au moins un parmi R⁵, R⁶ et R⁷ est R¹⁰-C(O)-NH-R¹¹-, dans lequel R¹⁰ est alkyle (C₁-C₅₀) ou alcényle (C₂-C₅₀), et R¹¹ est alkylène (C₁-C₂₀), typiquement méthylène ou polyméthylène (C₂-C₂₀).

8. Composition selon la revendication 1, dans laquelle au moins un parmi R⁵, R⁶ et R⁷ est un radical R¹⁰-C(O)-NH-R¹¹-, dans lequel R¹⁰ est alkyle (C₁-C₅₀) ou alcényle (C₂-C₅₀), et la partie R¹⁰-C(O)- du radical est dérivée de : huile d'amande, huile de babassu, huile de noyaux de palme, suif hydrogéné, lait, huile de vison, huile d'olive, huile de palme, huile de sésame, soja, suif ou huile de germe de blé, et R¹¹ est alkylène (C₁-C₂₀), typiquement méthylène ou polyméthylène (C₂-C₂₀).

9. Composition selon la revendication 1, dans laquelle deux parmi R⁵, R⁶ et R⁷ sont fusionnés pour former un cycle hétérocyclique saturé ou non saturé de 5 à 6 chaînons qui comprend l'atome azote de l'oxyde d'amine et un deuxième atome azote comme chaînons de cycle, et dans laquelle le cycle est substitué par amino sur un ou plusieurs des atomes du cycle.

10. Composition selon la revendication 1, dans laquelle deux parmi R⁵, R⁶ et R⁷ sont fusionnés pour former un cycle hétérocyclique saturé ou non saturé de 5 à 6 chaînons qui comprend l'atome azote de l'oxyde d'amine et un atome oxygène comme chaînons du cycle et dans laquelle le cycle est substitué par alkyle sur un ou plusieurs carbones du cycle.

11. Composition selon la revendication 1, dans laquelle la composition comprend en outre entre 0,5 et 30 parties en poids d'un ou plusieurs tensio-actifs sélectionnés parmi des tensio-actifs cationiques, tensio-actifs non ioniques, tensio-actifs amphotères et tensio-actifs zwitterioniques.

12. Procédé de production d'une composition de tensio-actif structuré aqueux comprenant le mélange, sur la base de 100 parties en poids de la composition :
de l'eau,
de plus de 0 à 6 parties en poids d'un électrolyte sélectionné parmi les pyrophosphate de potassium, tripolyphosphate de potassium, citrate de sodium, citrate de potassium, chlorure de calcium, bromure de calcium, halogénures de zinc, chlorure de baryum, nitrate de calcium, halogénures métalliques alcalins, halogénures d'ammonium, nitrate métallique alcalin, nitrates d'ammonium, polyacrylates non coiffés, polymaléates, polycarboxylates, lignosulphonates et copolymères de sulfonate de naphtalène et de formaldéhyde,
de 8 à 40 parties en poids d'un ou plusieurs tensio-actifs anioniques sélectionnés parmi les sulfonates d'alkylbenzène linéaires, sulfonates d'alpha-oléfine, sulfonates de paraffine, sulfonates d'esters alkyliques, sulfates d'alkyle, sulfates d'alkyle à liaison alkoxy, sulfonates d'alkyle, sulfates d'alkoxy à liaison alkyle, sulfates alcoxylés d'alkyle, phosphates monoalkyles, phosphates dialkyles, sarcosinates, sulfosuccinates, iséthionates et taurates, et leurs mélanges,
et de plus de 0 à 5 parties en poids d'un ou plusieurs oxydes d'amine selon la formule (III) : 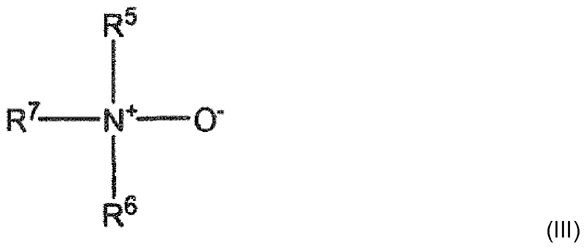
dans laquelle R⁵, R⁶ et R⁷ sont indépendamment les uns des autres alkyle, hydroxyalkyle, alkoxyle, alcényle, R⁸-R⁹-, R¹⁰-C(0)-NH-R¹¹-, une fraction phosphate, une fraction phosphonate, ou deux parmi R⁵, R⁶ et R⁷ sont fusionnés pour former un cycle hétérocyclique saturé ou non saturé de 5 à 8 chaînons qui comprend l'atome azote de l'oxyde d'amine et qui en outre comprend facultativement un deuxième atome azote ou un atome oxygène comme chaînon du cycle, et qui peut être en outre substitué par alkyle ou amino sur un ou plusieurs des atomes du cycle,
R⁸ et R¹⁰ sont indépendamment les uns des autres H, alkyle ou alcényle,
R⁹ est alkylèneoxy, et
R¹¹ est alkylène ou alkylèneoxy,
dans laquelle la quantité d'oxyde d'amine est de 10 à 50 parties en poids pour 100 parties en poids du tensio-actif anionique, dans laquelle la composition présente une viscosité rhéofluidifiante et est capable de mettre en suspension des composants insolubles ou partiellement solubles dans l'eau.

13. Composition multiphase comprenant :
une première phase qui comprend, sur la base de 100 parties en poids de la composition :
de l'eau,
de plus de 0 à 6 parties en poids d'un électrolyte sélectionné parmi les pyrophosphate de potassium, tripolyphosphate de potassium, citrate de sodium, citrate de potassium, chlorure de calcium, bromure de calcium, halogénures de zinc, chlorure de baryum, nitrate de calcium, halogénures métalliques alcalins, halogénures d'ammonium, nitrate métallique alcalin, nitrates d'ammonium, polyacrylates non coiffés, polymaléates, polycarboxylates, lignosulphonates et copolymères de sulfonate de naphtalène et de formaldéhyde,
de 8 à 40 parties en poids d'un ou plusieurs tensio-actifs anioniques sélectionnés parmi les sulfonates d'alkylbenzène linéaires, sulfonates d'alpha-oléfine, sulfonates de paraffine, sulfonates d'esters alkyliques, sulfates d'alkyle, sulfates d'alkyle à liaison alkoxy, sulfonates d'alkyle, sulfates d'alkoxy à liaison alkyle, sulfates alcoxylés d'alkyle, phosphates monoalkyles, phosphates dialkyles, sarcosinates, sulfosuccinates, iséthionates et taurates, et leurs mélanges,
et de plus de 0 à 5 parties en poids d'un ou plusieurs oxydes d'amine selon la formule (III) : 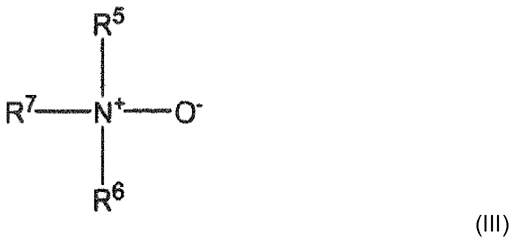
dans laquelle R⁵, R⁶ et R⁷ sont indépendamment les uns des autres alkyle, hydroxyalkyle, alkoxyle, alcényle, R⁸-R⁹-, R¹⁰-C(O)-NH-R¹¹-, une fraction phosphate, une fraction phosphonate, ou deux parmi R⁵, R⁶ et R⁷ sont fusionnés pour former un cycle hétérocyclique saturé ou non saturé de 5 à 8 chaînons qui comprend l'atome azote de l'oxyde d'amine et qui en outre comprend facultativement un deuxième atome azote ou un atome oxygène comme chaînon du cycle, et qui peut être en outre substitué par alkyle ou amino sur un ou plusieurs des atomes du cycle,
R⁸ et R¹⁰ sont indépendamment les uns des autres H, alkyle ou alcényle,
R⁹ est alkylèneoxy, et
R¹¹ est alkylène ou alkylèneoxy,
dans laquelle la quantité d'oxyde d'amine est de 10 à 50 parties en poids pour 100 parties en poids du tensio-actif anionique, qui présente une viscosité rhéofluidifiante et qui est capable de mettre en suspension des composants insolubles ou partiellement solubles dans l'eau, et
une ou plusieurs phases additionnelles qui comprennent un ou plusieurs composants insolubles ou partiellement solubles dans l'eau et qui sont au moins sensiblement distinctes de la première phase.

14. Composition selon la revendication 13, dans laquelle la composition est une composition de soins personnels et le ou les composants insolubles ou partiellement solubles dans l'eau comprennent un ou plusieurs agents traitants.

15. Composition selon la revendication 14, dans laquelle la composition est sélectionnée parmi des shampooings, produits de nettoyage du corps, savons pour les mains, lotions, crèmes, conditionneurs, produits de rasage, produits de nettoyage du visage, lingettes personnelles et traitements pour la peau.

16. Composition selon la revendication 14, dans laquelle la composition comprend en outre un ou plusieurs agents traitants comprenant un ou plusieurs composés sélectionnés parmi des émollients, hydratants, conditionneurs, conditionneurs pour la peau, conditionneurs pour les cheveux, vitamines, provitamines, antioxydants, phagocytes de radicaux libres, agents abrasifs, teintures, agents colorants pour cheveux, agents blanchissants, absorbeurs d'UV, agents anti-UV, agents antibactériens, agents antifongiques, régulateurs de mélanine, accélérateurs de bronzage, agents de dépigmentation, agents éclaircissant la peau, agents colorant la peau, liporégulateurs, agents de réduction du poids, agents anti-acné, agents antiséborrhéiques, agents antivieillissement, agents antirides, agents kératolytiques, agents anti-inflammatoires, agents anti-acné, antibiotiques, agents anti-inflammatoires, extraits botaniques, imidazoles, agents rafraichissants, agents cicatrisants, agents de protection vasculaire, agents de réduction des pellicules, de la dermatite séborrhéique ou du psoriasis, huile de schiste et ses dérivés, agents anti-psoriasis, corticostéroïdes, agents de dépilation, agents pour combattre la chute des cheveux, réducteurs pour permanentes, agents réfléchissants, huiles essentielles et fragrances.
